Europäisches Patentamt

European Patent Office

Office européen des brevets

(19)

(11) EP 0 930 503 B1

(12) **FASCICULE DE BREVET EUROPEEN**

(45) Date de publication et mention
de la délivrance du brevet:
**25.04.2001 Bulletin 2001/17**

(51) Int Cl.⁷: **G01N 33/28**, G01N 7/18

(21) Numéro de dépôt: **99400075.0**

(22) Date de dépôt: **14.01.1999**

(54) **Méthode de caractérisation de la capacité de neutralisation d'un lubrifiant et dispositif pour la mise en oeuvre de cette méthode**

Verfahren zur Kennzeichnung der Neutralisierungskapazität eines Schmiermittels und Vorrichtung zur Durchführung dieses Verfahrens

Method for characterizing the neutralizing capacity of a lubricant and apparatus for carrying out this method

(84) Etats contractants désignés:
**DE DK ES FR GB IT NL SE**

(30) Priorité: **16.01.1998 FR 9800457**

(43) Date de publication de la demande:
**21.07.1999 Bulletin 1999/29**

(73) Titulaire: **ELF ANTAR FRANCE**
**92400 Courbevoie (FR)**

(72) Inventeur: **Roman, Jean-Philippe**
**F-69001 Lyon (FR)**

(74) Mandataire: **Timoney, Ian Charles Craig**
**Elf Exploration Production**
**Département Propriété Industrielle**
**Tour Elf**
**EP/T/RD/DPI - Bureau 34 G 47**
**92078 Paris La Défense Cedex (FR)**

(56) Documents cités:
**DD-A- 217 895          FR-A- 2 141 414**
**FR-A- 2 486 246**

• **PATENT ABSTRACTS OF JAPAN vol. 13, no. 175 (P-863), 25 avril 1989 & JP 01 009364 A (TATSUTA ELECTRIC WIRE & CABLE CO LTD), 21 janvier 1989**

Printed by Jouve, 75001 PARIS (FR)

## Description

DOMAINE TECHNIQUE

La présente invention concerne une méthode de caractérisation de la capacité de neutralisation d'un lubrifiant contenant des additifs détergents renfermant une réserve de basicité ainsi qu'un dispositif pour la mise en oeuvre de cette méthode.

Elle trouve son application dans les laboratoires de recherche et de contrôle des fabricants de lubrifiants ainsi que dans ceux des motoristes.

ETAT DE LA TECHNIQUE ANTERIEURE

Les moteurs diesels et plus particulièrement les moteurs diesels marins de grosse taille utilisent généralement comme carburants des fiouls lourds à forte teneur en soufre. Lors de la combustion, le soufre peut conduire à la formation d'acide sulfurique sous l'effet de réactions d'oxydation et par combinaison avec de l'eau.

Cet acide qui a tendance à condenser à l'intérieur des moteurs diesels peut entraîner la corrosion des pièces métalliques et l'usure corrosive d'organes majeurs comme les chemises et les segments.

Pour minimiser ces effets nuisibles les lubrifiants utilisés pour ces moteurs doivent présenter une capacité de neutralisation.

Pour cette raison ces lubrifiants sont des produits à forte basicité.

Cette basicité est habituellement apportée par des additifs détergents surbasés qui renferment une espèce neutralisante, par exemple du carbonate de calcium.

Pour mesurer cette basicité on utilise couramment une méthode décrite dans la norme ASTM D-2896, qui permet de déterminer un paramètre appelé BN (Basic Number en anglais) qui s'exprime en mg d'hydroxyde de potassium (KOH) par gramme de lubrifiant.

Ce paramètre est un indicateur macroscopique, qui permet de classer les lubrifiants en fonction du potentiel nécessaire pour assurer une protection suffisante et durable des moteurs vis à vis des phénomènes indésirables liés à la présence d'acides en général.

Ce paramètre représentatif de la basicité du lubrifiant ne prend pas en compte les conditions réelles d'oxydation des lubrifiants dans les moteurs diesels il ne peut donc pas caractériser complètement leur capacité de neutralisation.

Une autre méthode de caractérisation de la capacité de neutralisation des lubrifiants est décrite dans le document Technical Papers Series, n° 872 158 intitulé "Cylinder Wear Mechanism in an EGR-Equiped Diesel Engine and Wear Protection by the Engine Oil" publié par Society of Automotive Engineers.

Cette méthode vise à mesurer la vitesse de neutralisation du lubrifiant par l'acide sulfurique. Elle consiste à introduire un échantillon de 30 grammes de lubrifiant dans un réservoir étanche muni d'un thermomètre et d'un manomètre et comportant un évent.

Le réservoir étant immergé dans un bain d'eau à température constante et l'échantillon de lubrifiant étant constamment agité par des moyens adaptés, lorsque la température de l'échantillon est stabilisée, 0,1 millilitre d'acide sulfurique concentré sont introduits à l'intérieur du réservoir au moyen d'une seringue à travers l'évent ouvert.

L'évent est ensuite immédiatement fermé.

A partir de cet instant la pression indiquée par le manomètre est enregistrée pendant 10 minutes.

Les valeurs de la pression enregistrée sont converties en quantité de dioxyde de carbone produit par la réaction de l'acide sulfurique avec les produits basiques contenus dans l'échantillon de lubrifiant.

La vitesse de neutralisation est caractérisée par le volume de dioxyde de carbone produit au cours des 4 minutes qui suivent l'introduction de l'acide, elle s'exprime en micromoles de dioxyde de carbone.

Cette méthode présente l'inconvénient de mettre en oeuvre un volume important de lubrifiant alors que au contraire dans un moteur en fonctionnement les phénomènes de neutralisation se produisent à la surface des organes lubrifiés tels que les cylindres et pistons et les quantités de lubrifiants mises en jeu sont faibles.

Avec cette méthode la mesure de la vitesse de neutralisation est très dépendante des conditions d'agitation du lubrifiant dans le réservoir à l'intérieur duquel s'effectue la réaction ce qui est préjudiciable à répétabilité de la mesure et à la validité des comparaisons entre des mesures effectuées avec différents lubrifiants.

De plus, elle n'est représentative que des phénomènes lents c'est à dire dont la durée est de plusieurs minutes.

EXPOSE DE L'INVENTION

La présente invention a justement pour objet de remédier à ces inconvénients et notamment de fournir un procédé et un dispositif de caractérisation de la capacité de neutralisation des lubrifiants qui prend en compte les conditions réelles d'oxydation des lubrifiants dans les moteurs. En effet les résultats obtenus par la méthode de l'invention sont représentatifs des phénomènes de neutralisation qui se produisent essentiellement à la surface des organes lubrifiés qui sont caractérisés par les faibles quantités de lubrifiants mises en jeu instantanément et les fortes concentrations locales en acide qui résultent des phénomènes de condensation et de solubilisation de l'acide dans le lubrifiant.

Elle trouve son application dans les laboratoires des industries qui fabriquent des lubrifiants et ceux des constructeurs de moteurs auxquels ces lubrifiants sont destinés.

A cette fin la présente invention propose une méthode de caractérisation de la capacité de neutralisation d'un lubrifiant contenant des additifs détergents renfermant une réserve de basicité, consistant à faire réagir, dans un réservoir étanche, à température constante les bases apportées par les additifs contenus dans un échantillon de masse connue de lubrifiant avec une quantité déterminée d'un acide liquide, au moins égale à la quantité stoechiométrique, caractérisée en ce qu'elle consiste en outre :

- à former à l'intérieur du réservoir étanche un film à partir de l'échantillon de lubrifiant,
- à déposer la quantité déterminée d'acide liquide à la surface du film dans un temps inférieur à 0,5 seconde,
- à enregistrer en fonction du temps les valeurs de la pression à l'intérieur du réservoir étanche jusqu'à stabilisation de ladite pression,
- à déterminer à partir des valeurs enregistrées de la pression, au moins un des trois paramètres caractérisants suivants : un délai d'initiation de la réaction, un potentiel de neutralisation, une vitesse de réaction.

Selon une deuxième caractéristique de la méthode de l'invention, le délai d'initiation de la réaction est déterminé par la mesure du temps qui s'écoule entre le début du dépôt de l'acide liquide et l'instant de passage par une valeur minimale de la pression enregistrée.

Selon une autre caractéristique de la méthode de l'invention la quantité de bases apportées par les additifs détergents contenus dans l'échantillon de lubrifiant étant connue, la pression enregistrée atteignant une valeur stable, le potentiel de neutralisation est déterminé par application de la formule suivante :

$$Ra = \frac{\Delta P}{Pth} \times 100$$

dans laquelle

Ra   représente le potentiel de neutralisation en %,

$\Delta P$   représente la différence entre la valeur de pression enregistrée stabilisée et la valeur minimale de la pression enregistrée,

Pth   représente la pression théorique dans le réservoir étanche, qui aurait été atteinte si la réaction avait été complète.

Selon une autre caractéristique de la méthode de l'invention la vitesse de réaction est égale à la vitesse maximale d'augmentation de la pression enregistrée.

Selon une autre caractéristique de la méthode de l'invention le film de lubrifiant formé à l'intérieur du réservoir étanche présente une épaisseur comprise entre 20 et 150 microns.

Selon une autre caractéristique de la méthode de l'invention l'acide liquide est préférentiellement de l'acide sulfurique à 95%.

Selon une autre caractéristique de la méthode de l'invention la quantité déterminée d'acide sulfurique à 95%, correspond à un excès d'acide compris entre 0,5% et 200 % et préférentiellement égal à 96,7%, par rapport à la quantité de bases apportée par les additifs détergents.

L'invention a aussi pour objet un dispositif pour la caractérisation de la capacité de neutralisation d'un lubrifiant contenant des additifs détergents renfermant une réserve de basicité, comportant un réservoir étanche muni de moyens de maintien à une température constante, dans lequel on fait réagir les bases apportées par les additifs contenus dans un échantillon de masse connue de lubrifiant avec une quantité déterminée d'un acide liquide, au moins égale à la quantité stoechiométrique, caractérisé en ce qu'il comporte en plus :

- des moyens de formation d'un film, sur une paroi interne du réservoir étanche à partir de l'échantillon de lubrifiant,
- des moyens de dépôt de la quantité déterminée d'acide liquide à la surface du film dans un temps inférieur à 0,5 seconde,
- un capteur de mesure de pression à l'intérieur du réservoir étanche délivrant un signal de mesure de pression sur une sortie,
- un module d'enregistrement relié à la sortie du capteur de pression pour enregistrer en fonction du temps les valeurs de la pression à l'intérieur du réservoir étanche,
- des moyens de détermination d'au moins un des trois paramètres caractérisants suivants : un délai d'initiation de la réaction, un potentiel de neutralisation, une vitesse de réaction.

Selon une autre caractéristique du dispositif de l'invention les moyens de détermination des paramètres caractérisants consistent en un module de visualisation d'une courbe représentative de la valeur de la pression enregistrée en fonction du temps, dans un système d'axes gradués, ledit module étant raccordé à une sortie du module d'enregistrement.

Selon une autre caractéristique du dispositif de l'invention le délai d'initiation de la réaction est déterminé à partir de la courbe représentative de la valeur de la pression enregistrée, par la mesure du temps qui s'écoule entre le début du dépôt de l'acide liquide et l'instant de passage par une valeur minimale de la pression enregistrée.

Selon une autre caractéristique du dispositif de l'invention la quantité de bases apportées par les additifs détergents contenus dans l'échantillon de lubrifiant étant connue, la pression enregistrée atteignant une valeur stable, le potentiel de neutralisation est déterminé par application de la formule suivante :

$$Ra = \frac{\Delta P}{Pth} \times 100$$

dans laquelle :

Ra  représente le potentiel de neutralisation en %,

$\Delta P$  représente la différence entre la valeur de pression enregistrée stabilisée et la valeur minimale de la pression enregistrée,

Pth  représente la pression théorique dans le réservoir étanche, qui aurait été atteinte si la réaction avait été complète,

$\Delta P$  est déterminée à partir de la courbe représentative de la valeur de la pression enregistrée.

Selon une autre caractéristique du dispositif de l'invention la vitesse de réaction, égale à la vitesse maximale d'augmentation de la pression enregistrée est déterminée à partir de la courbe représentative de la valeur de la pression enregistrée.

Selon une autre caractéristique le dispositif de l'invention comporte en plus un indicateur de début du dépôt d'acide liquide qui délivre sur une sortie un signal de début de dépôt d'acide liquide, reliée au module d'enregistrement et en ce que les moyens de détermination des paramètres caractérisants consistent un module électronique de calcul desdits paramètres, raccordé à une sortie du module d'enregistrement

Selon une autre caractéristique du dispositif de l'invention le module électronique de calcul détermine le délai d'initiation de la réaction à partir de la valeur de la pression enregistrée et du signal délivré par l'indicateur de début du dépôt d'acide liquide, par le calcul du temps qui s'écoule entre le début du dépôt de l'acide liquide et l'instant de passage par une valeur minimale de la pression enregistrée.

Selon une autre caractéristique du dispositif de l'invention la quantité de bases apportées par les additifs détergents contenus dans l'échantillon de lubrifiant étant connue, la pression enregistrée atteignant une valeur stable, le potentiel de neutralisation est déterminé par application de la formule suivante :

$$Ra = \frac{\Delta P}{Pth} \times 100$$

dans laquelle :

Ra  représente le potentiel de neutralisation en %,

$\Delta P$  représente la différence entre la valeur de pression enregistrée stabilisée et la valeur minimale de la pression enregistrée,

Pth  représente la pression théorique dans le réservoir étanche, qui aurait été atteinte si la réaction avait été complète,

$\Delta P$  est calculée par le module électronique de calcul à partir des valeurs de la pression enregistrée.

Selon une dernière caractéristique du dispositif de l'invention le module électronique de calcul détermine la vitesse de réaction, à partir des valeurs de la pression enregistrée, par le calcul de la vitesse maximale d'augmentation de la pression enregistrée.

<u>BREVE DESCRIPTION DES DESSINS</u>

L'invention sera mieux comprise à l'aide de la description qui suit, de deux modes de réalisation donnés à titre d'exemples et en référence aux dessins annexés dans lesquels :

- la figure 1 représente schématiquement un premier mode de réalisation du dispositif de l'invention comportant un traceur.
- la figure 2 est une vue en coupe partielle qui montre les positions respectives du corps du réservoir étanche et des barreaux aimantés du dispositif de l'invention.
- la figure 3 représente une courbe de variation de la pression à l'intérieur du réservoir du dispositif de l'invention en fonction du temps,
- la figure 4 représente schématiquement un deuxième mode de réalisation du dispositif de l'invention qui comporte un module électronique de calcul.
- la figure 5 représente la courbe de variation de la pression à l'intérieur du réservoir du dispositif de l'invention en fonction du temps obtenue avec un lubrifiant présentant un BN de 70 mg KOH/g.

<u>EXPOSE DETAILLE DE L'INVENTION</u>

D'une manière générale, la méthode et le dispositif de l'invention sont utilisés pour caractériser la capacité de neutralisation d'un lubrifiant contenant des additifs détergents renfermant une réserve de basicité.

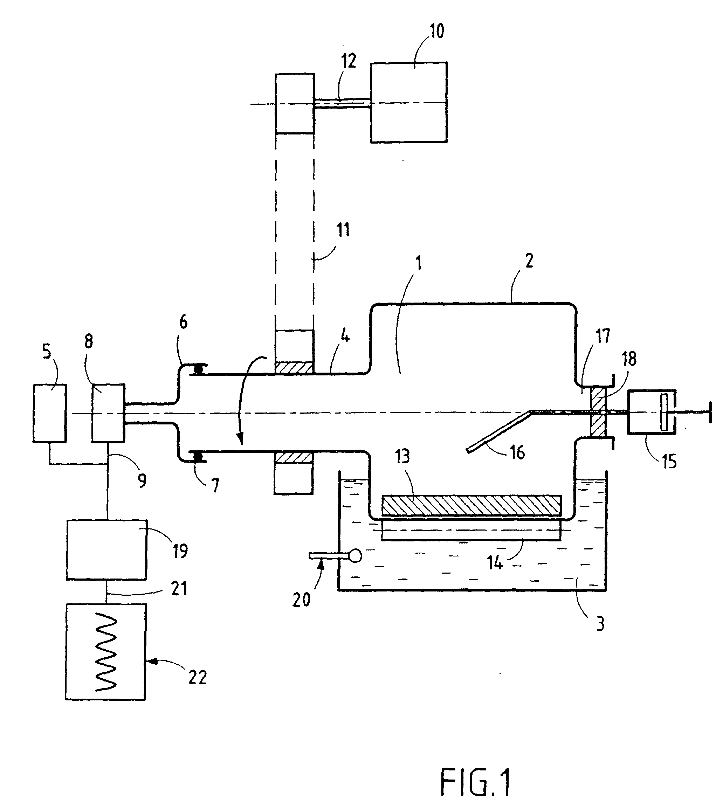

La figure 1 représente schématiquement un premier mode de réalisation du dispositif de l'invention, pour déterminer des paramètres caractérisants la capacité de neutralisation de lubrifiants à partir de résultats de mesures visualisés sur une table traçante.

Le dispositif selon ce premier mode réalisation comporte :

- un réservoir 1 étanche, transparent, de forme cylindrique, à axe horizontal constitué d'un corps 2, prolongé par un col 4 de plus petit diamètre fermé par un bouchon 6. L'étanchéité entre le col 4 et le bouchon 6 étant assurée par un joint torique 7.
- un capteur 8 de pression à réponse rapide monté de manière étanche sur le bouchon 6, qui délivre sur une sortie 9 un signal représentatif de la pression à l'intérieur du réservoir 1 étanche.
- un indicateur 5 de pression relié à la sortie 9 du capteur 8 de pression
- un enregistreur 19 rapide relié électriquement à la sortie 9 du capteur 8 de pression,
- un traceur 22 de courbe relié à une sortie 21 de l'enregistreur 19,
- un moteur 10 électrique muni d'un axe 12 qui entraîne en rotation autour de son axe horizontal le corps 2 du réservoir 1 grâce à des moyens 11 de liaison.

- un bain 3 thermostaté dans lequel la partie inférieure du corps 2 du réservoir 1 est partiellement immergée, dont la température est mesurée par un thermomètre 20 et régulée par des moyens adaptés non représentés sur la figure 1,

- une seringue 15 munie d'une aiguille 16 de forme incurvée à son extrémité libre.

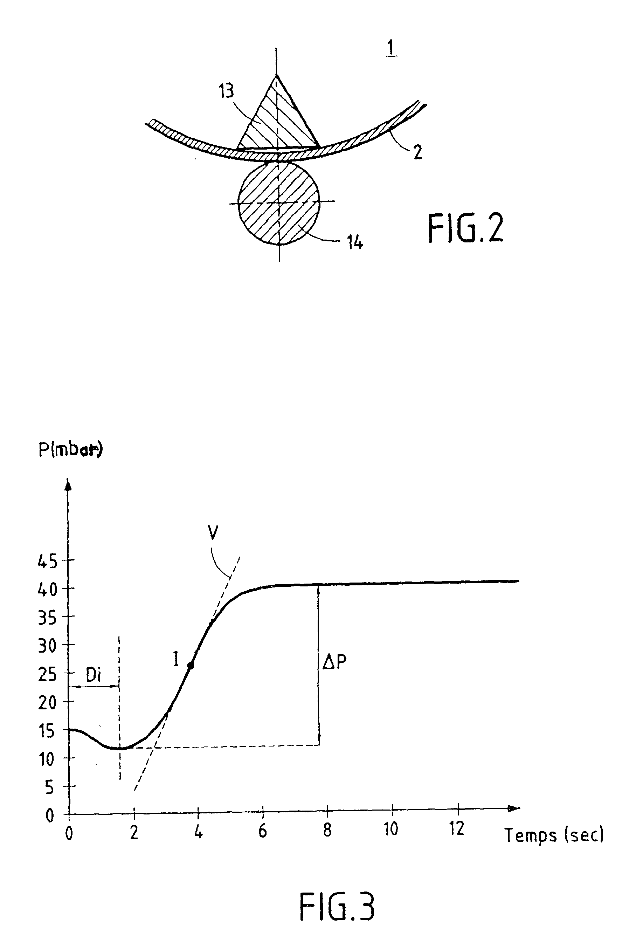

En plus le dispositif de l'invention comporte, placé à l'intérieur du corps 2 un premier barreau 13 aimanté, cylindrique dont la section à la forme d'un triangle équilatéral de hauteur sensiblement égale à 0,08 fois le diamètre du corps 2, de longueur un peu inférieure à la longueur d'une génératrice du corps 2 et placé à l'extérieur du corps 2 un deuxième barreau 14 aimanté cylindrique de section circulaire de diamètre comparable à la hauteur du premier barreau aimanté. Ces deux barreaux 13 et 14 aimantés étant disposés en regard l'un de l'autre à la partie basse du corps 2 de manière à ce qu'ils s'attirent mutuellement sous l'effet de leur champ magnétique. Le détail des positions relatives du corps 2 et des barreaux aimantés 13 et 14 est représenté sur la figure 2.

Le corps 2 du réservoir 1 étanche comporte sur la face opposée au col 4 une ouverture 17 axiale, fermée par un septum 18.

En agissant sur les moyens de régulation de la température du bain 3 thermostaté on fixe la valeur à laquelle on veut opérer, par exemple 80°C. Lorsque cette température mesurée par le thermomètre 20 est atteinte et stabilisée, on retire le septum 18 et on introduit par l'ouverture 17 à l'intérieur du corps 2 du réservoir 1, une quantité déterminée du lubrifiant dont on veut caractériser la capacité de neutralisation puis on remet en place le septum 18.

La quantité déterminée de lubrifiant est calculée à partir des dimensions du corps 2 du réservoir et de l'épaisseur du film qu'on veut former.

On alimente le moteur 10 électrique qui met en rotation le réservoir 1 par l'intermédiaire des moyens 11 d'entraînement qui relient l'axe 12 du moteur au réservoir 1. La vitesse de rotation du réservoir est par exemple de 1,5 tour par seconde.

Les barreaux 13 et 14 aimantés coopèrent avec la paroi de la partie inférieure du réservoir 1 pour former un film de lubrifiant d'épaisseur comprise entre 20 et 150 microns. Après quelques minutes de rotation du réservoir 1, on vérifie visuellement que le film formé est homogène, sa température est alors très voisine de celle du bain thermostaté.

Cet état est atteint lorsque la pression à l'intérieur du réservoir 1, indiquée par l'indicateur 5 de pression ne varie plus.

On introduit alors manuellement l'aiguille 16 de la seringue 15 préalablement remplie d'un acide liquide, à l'intérieur du réservoir 1 au travers du septum 18 de manière à ce que son extrémité libre incurvée soit dirigée vers la partie inférieure du corps 2 du réservoir 1 immergée dans le bain 3 thermostaté. En agissant manuellement sur le piston de la seringue 15 on expulse dans un temps de l'ordre de 0,5 seconde une quantité d'acide sulfurique au moins égale à la quantité stoechiométrique pour réagir avec les éléments basiques apportés par les additifs contenus dans le film de lubrifiant. Pour faciliter cette opération on peut arrêter le moteur 10 pendant un instant très court. Cette quantité d'acide ainsi introduite dans le réservoir 1 se dépose sur le film de lubrifiant avec lequel elle réagit.

La réaction acido-basique produit un dégagement de dioxyde de carbone qui a pour effet d'augmenter la pression dans le réservoir 1 étanche.

Simultanément au début du dépôt d'acide on démarre l'enregistrement de la pression à l'intérieur du réservoir 1. On poursuit cet enregistrement jusqu'à ce que la pression ne varie plus c'est à dire pendant environ 10 secondes.

On obtient ainsi sur le traceur 22 la courbe de variation de la pression en fonction du temps dont un exemple est donné figure 3.

Sur cette courbe on peut distinguer trois zones correspondantes aux trois phases de la réaction : la phase d'initiation qui correspond à la solubilisation de l'acide et à sa diffusion, la phase de réaction proprement dite et la phase d'arrêt ou de limitation de la réaction.

A partir de cette courbe on détermine les trois paramètres caractérisants :

- le délai d'initiation de la réaction donné par la mesure du temps qui s'écoule entre le début de l'introduction de l'acide dans le réservoir 1 et l'instant de passage de la pression à sa valeur minimale Il correspond à l'intervalle de temps repéré Di sur la figure 3.

- le potentiel de neutralisation

On observe que la pression à l'intérieur du réservoir se stabilise à une valeur inférieure à la pression théorique qui aurait été atteinte si toute la base disponible dans le film avait réagi qu'on peut calculer au moyen de la formule connue donnée ci-après.

La réaction acido-basique n'est donc pas totale. Ce résultat est tout à fait surprenant puisque l'acide introduit dans le réservoir étant en excès toutes les bases contenus dans le film auraient du réagir.

On définit le potentiel de neutralisation par la formule suivante :

$$Ra = \frac{\Delta P}{Pth} \times 100$$

dans laquelle :

Ra représente le potentiel de neutralisation en %,

ΔP est égale à la différence entre la valeur de pression enregistrée stabilisée et la valeur minimale de la pression enregistrée repérée Δ P sur la figure 3,

Pth représente la pression théorique du dioxyde de carbone dans le réservoir étanche, qui aurait été atteinte si la réaction avait été complète.

On calcule Pth à partir de la formule suivante connue :

$$Pth = Nth.R.T / V$$

dans laquelle :

Pth est exprimée en Pascal

Nth est le nombre théorique de moles de dioxyde carbone qui auraient été produites si la réaction avait été complète,

R est la constante des gaz parfaits égale à 8,32 Joule

V représente le volume du réservoir exprimé en mètre cube

T représente la température du bain thermostaté exprimée en Kelvin

Nth est calculé à partir, du BN (Basic Number) du lubrifiant déterminé préalablement selon la méthode décrite dans la norme ASTM - D 2896, de la masse du lubrifiant introduite dans le réservoir pour former le film et de l'acide utilisé. BN s'exprime en mg d'hydroxyde de potassium par gramme de lubrifiant.

- La vitesse de réaction

Elle est égale à la vitesse maximale de variation de la pression enregistrée. Elle correspond à la pente de la courbe de la figure 3 calculée au point d'inflexion repéré I. Elle est caractéristique d'une cinétique d'ordre 1 et représente physiquement le phénomène de neutralisation du lubrifiant sous la forme d'un film mince.

L'un des principaux avantages de la méthode de l'invention est qu'elle prend en compte séparément les phénomènes rapides qui se produisent dans les secondes qui suivent l'introduction d'acide dans le réservoir étanche.

Les trois paramètres ainsi déterminés dans des conditions proche de celles dans lesquelles un lubrifiant doit être utilisé, caractérisent de façon précise sa capacité de neutralisation.

EXEMPLE :

Caractéristiques principales du réservoir étanche :

- nature : verre transparent

- diamètre du corps 2 : 100 mm

- longueur du corps 2 : 65mm

- diamètre du col : 40 mm

- volume total V : $7,5.10^{-4}$ m$^3$

Caractéristiques des barreaux aimantés

- hauteur du premier barreau 13 : 11 mm

- hauteur du deuxième barreau 14 : 11 mm

Capteur de pression :

- type: ELESTA CP 1100

- sensibilité : 0,02 mbar

- échelle: 0 à 100 mbar

- temps de réponse : 100 ms

Enregistreur - table traçante :

- sensibilité : 50 ms
- vitesse de déroulement : 0,25 cm/s

Vitesse de rotation du réservoir : 1,5 tour par seconde

Température du bain thermostaté : 80 °C soit 353 K

Données relatives au lubrifiant :

- type : pour moteur marin
- BN selon norme ASTM - D 2896 : 70 mg KOH / g de lubrifiant
- quantité introduite dans le réservoir pour former le film : 2 g
- espèce neutralisante : carbonate de calcium (CaCO3)

Données relatives à l'acide liquide :

- nature : acide sulfurique à 95%
- quantité déposée sur le film : 2 ml
- excès calculé par rapport à la stoechiométrie : 96,7 %

Calcul de la valeur de Pth :

$$Pth = Nth.R.T / V$$

Nombre de moles de KOH dans le lubrifiant :
masse de KOH / masse molaire KOH
masse lubrifiant x BN / masse molaire KOH
soit $2 \times 70 \times 10^{-3} / 56 = 0,0025$ mole de KOH
d'où $Nth = 0,0025 / 2 = 0,00125$ mole de $CaCO_3$
$R = 8,32$ Joule, $T = 273 + 80 = 353$ K, $V = 7,5$ $10^{-4}$ $m^3$
$Pth = 1,25 . 10^{-3} \times 8,32 \times 353 / 7,5 . 10^{-4} = 4895$ Pa soit 48,95 mbar

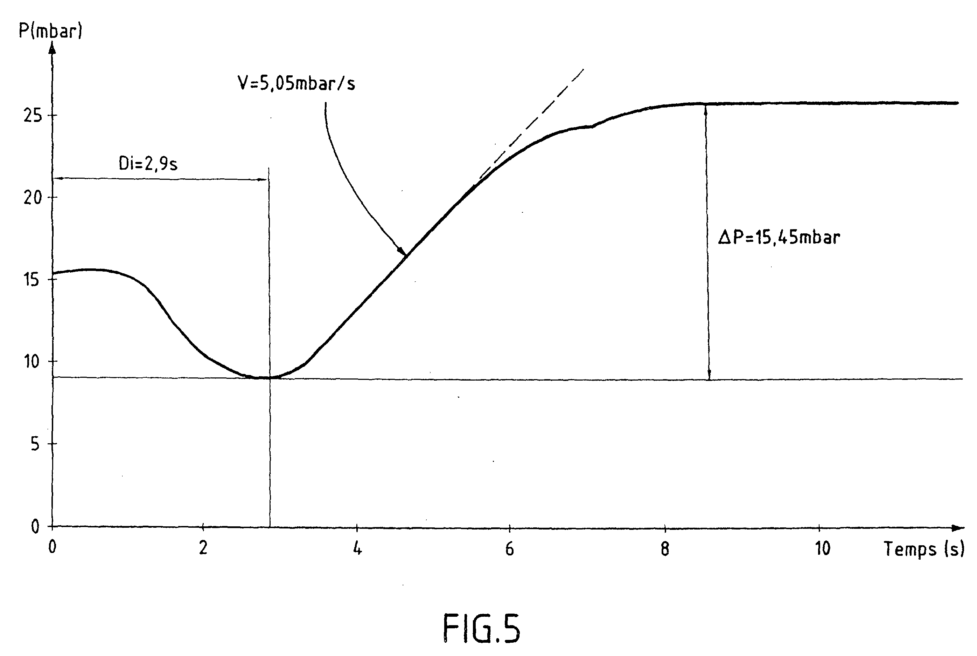

La courbe obtenue est celle de la figure 5 à partir de laquelle on détermine les paramètres caractérisants la capacité de neutralisation du lubrifiant indiqués ci-après :

- délai d'initiation (mesuré sur la figure 5) : 2,9 secondes
- potentiel de neutralisation :
à partir de la valeur de $\Delta P$ mesurée sur la figure 5 soit 15,4 mbar et de Pth on calcule Ra par application de la formule $Ra = (\Delta P/Pth) \times 100$ soit $Ra = (15,4/48,9) \times 100 = 31,6\%$
- vitesse de réaction (mesurée sur la figure 5) : 5,05 mbar / seconde

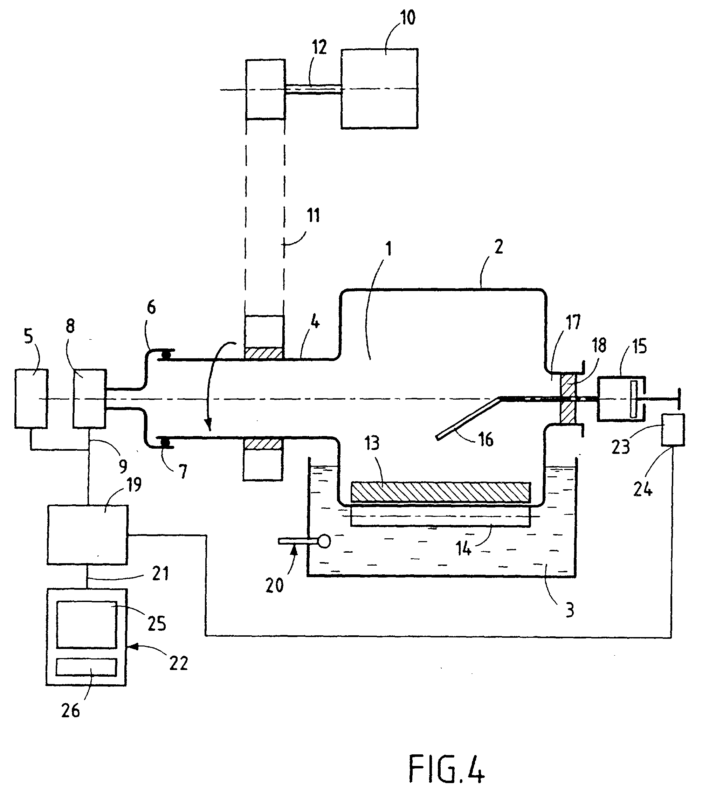

Selon un deuxième mode de réalisation représenté schématiquement sur la figure 4 le dispositif de l'invention comporte :

- un réservoir 1 étanche, transparent, de forme cylindrique, à axe horizontal constitué d'un corps 2, prolongé par un col 4 de plus petit diamètre fermé par un bouchon 6. L'étanchéité entre le col 4 et le bouchon 6 étant assurée par un joint torique 7.
- un capteur 8 de pression à réponse rapide monté de manière étanche sur le bouchon 6, qui délivre sur une sortie 9 un signal représentatif de la pression à l'intérieur du réservoir 1 étanche.
- un indicateur 5 de pression relié à la sortie 9 du capteur 8 de pression,
- un microcontact 23 indicateur de début du dépôt d'acide ,
- un enregistreur 19 relié électriquement à la sortie 9 du capteur 8 de pression et à une sortie 24 de l'indicateur 23 de début du dépôt d'acide,
- un module 25 électronique de calcul relié à une sortie 21 de l'enregistreur 19 comportant : un indicateur 26 numérique à cristaux liquides et non représentés sur la figure 4 une unité de traitement, une mémoire et un clavier numérique pour introduire des données dans la mémoire,
- un moteur 10 électrique muni d'un axe 12 qui entraîne en rotation autour de son axe horizontal le corps 2 du réservoir 1 grâce à des moyens 11 de liaison.
- un bain 3 thermostaté dans lequel la partie inférieure du corps 2 du réservoir 1 est partiellement immergée, dont la température est mesurée par un thermomètre 20 et régulée par des moyens adaptés non représentés sur la figure 4

En plus le dispositif de l'invention comporte, placé à l'intérieur du corps 2 un premier barreau 13 aimanté, cylindrique dont la section à la forme d'un triangle équilatéral de hauteur sensiblement égale à 0,08 fois le diamètre du corps 2, de longueur un peu inférieure à la longueur d'une génératrice du corps 2 et placé à l'extérieur du corps 2 un deuxième barreau 14 aimanté cylindrique de section circulaire de diamètre comparable à la hauteur du premier barreau aimanté. Ces deux barreaux 13 et 14 aimantés étant disposés en regard l'un de l'autre à la partie basse du corps 2 de manière à ce qu'ils s'attirent mutuellement sous l'effet de leur champ magnétique. Le détail des positions relatives du corps 2 et des barreaux aimantés 13 et 14 est représenté sur la figure 2.

Le corps 2 du réservoir 1 étanche comporte sur la face opposée au col 4 une ouverture 17 axiale, fermée par un septum 18 lui même traversé par une aiguille 16 d'une seringue 15. Cette aiguille 16 de forme incurvée a son extrémité libre dirigée vers la partie basse du corps 2 immergée dans le bain thermostaté.

En agissant sur les moyens de régulation de la température du bain 3 thermostaté on fixe la valeur à laquelle on veut opérer, par exemple 80°C. Lorsque cette température mesurée par le thermomètre 20 est atteinte et stabilisée, on retire le septum 18 et on introduit par l'ouverture 17 à l'intérieur du corps 2 du réservoir 1, une quantité déterminée du lubrifiant dont on veut caractériser la capacité de neutralisation puis on remet en place le septum 18.

La quantité déterminée de lubrifiant est calculée à partir des dimensions du corps 2 du réservoir et de l'épaisseur du film qu'on veut former.

On alimente le moteur 10 électrique qui met en rotation le réservoir 1 par l'intermédiaire des moyens 11 d'entraînement qui relient l'axe 12 du moteur au réservoir 1. La vitesse de rotation du réservoir est par exemple de 1,5 tour par seconde.

Les barreaux 13 et 14 aimantés coopèrent avec la paroi de la partie inférieure du réservoir 1 pour former un film de lubrifiant d'épaisseur comprise entre 20 et 150 microns. Après quelques minutes de lubrifiant on vérifie visuellement que le film formé est homogène, sa température est alors très voisine de celle du bain thermostaté.

Cet état est atteint lorsque la pression à l'intérieur du réservoir 1, indiquée par l'indicateur 5 de pression ne varie plus.

On introduit alors manuellement l'aiguille 16 de la seringue 15 préalablement remplie d'un acide liquide, à l'intérieur du réservoir 1 au travers du septum. En agissant soit manuellement soit par l'intermédiaire d'un micro-vérin sur le piston de la seringue 15 on expulse dans un temps de l'ordre de 0,5 seconde une quantité d'acide sulfurique au moins égale à la quantité stoechiométrique pour réagir avec les éléments basiques apportés par les additifs contenus dans le film de lubrifiant. Pour faciliter cette opération on peut arrêter le moteur 10 pendant un instant très court. Cette quantité d'acide ainsi introduite dans le réservoir 2 se dépose sur le film de lubrifiant avec lequel elle réagit.

A l'instant du début de l'action sur le piston de la seringue 15 le micro-contact 23 est actionné et délivre un signal électrique sur sa sortie 24 raccordée à une entrée de l'enregistreur 19.

La réaction acide / base produit un dégagement de dioxyde de carbone qui a pour effet d'augmenter la pression dans le réservoir 1 étanche.

A l'instant d'apparition du signal délivré par le micro-contact l'enregistreur 19 commence à enregistrer la pression à l'intérieur du réservoir 1. Cet enregistrement se poursuit automatiquement pendant une durée au moins égale à 60 secondes.

Les valeurs de la pression enregistrées par l'enregistreur 19 sont transmises au module 25 électronique de calcul.

A partir de ces valeurs le module 25 électronique exécute un programme mémorisé dans sa mémoire pour calculer les trois paramètres caractérisant :

- le délai d'initiation de la réaction donné par la mesure du temps qui s'écoule entre l'instant d'apparition du signal délivré par l'indicateur 23 de début de l'introduction de l'acide dans le réservoir 1 et l'instant de passage de la pression à sa valeur minimale

- le potentiel de neutralisation défini par la formule suivante :

$$Ra = \frac{\Delta P}{Pth} \times 100$$

dans laquelle :

Ra représente le potentiel de neutralisation en %,
$\Delta P$ est égale à la différence entre la valeur de pression enregistrée stabilisée et la valeur minimale de la pression enregistrée,
Pth représente la pression théorique du dioxyde de carbone dans le réservoir étanche, qui aurait été atteinte si la réaction avait été complète.
Pth est calculé par le module électronique de calcul par application de la formule suivante :

$$Pth = Nth.R.T / V$$

dans laquelle :

Pth est exprimée en Pascal
Nth est le nombre théorique de moles de dioxyde carbone qui auraient été produites si la réaction avait été complète,
R est la constante des gaz parfaits égale à 8,32 Joule
V représente le volume du réservoir exprimé en mètre cube
T représente la température du bain thermostaté exprimée en Kelvin

Les valeurs de Pth, Nth, V, T, et R sont préalablement calculées et introduites manuellement dans la mémoire du module électronique de calcul au moyen du clavier numérique.

Nth est calculé à partir, du BN (Basic Number) du lubrifiant déterminé préalablement selon la méthode décrite dans la norme ASTM - D 2896, de la masse du lubrifiant introduite dans le réservoir pour former le film et de l'acide utilisé. BN s'exprime en mg d'hydroxyde de potassium par gramme de lubrifiant.

- La vitesse de réaction qui est égale à la vitesse maximale de variation de la pression enregistrée.

Les valeurs des trois paramètres ainsi calculées sont affichées sur l'écran 26 à cristaux liquides.

**Revendications**

1. Méthode de caractérisation de la capacité de neutralisation d'un lubrifiant contenant des additifs détergents renfermant une réserve de basicité, consistant à faire réagir, dans un réservoir (1) étanche, à température constante les bases apportées par les additifs contenus dans un échantillon de masse connue de lubrifiant avec une quantité déterminée d'un acide liquide, au moins égale à la quantité stoechiométrique, caractérisée en ce qu'elle consiste en outre :

- à former à l'intérieur du réservoir (1) étanche un film à partir de l'échantillon de lubrifiant,
- à déposer la quantité déterminée d'acide liquide à la surface du film dans un temps inférieur à 0,5 seconde,
- à enregistrer en fonction du temps les valeurs de la pression à l'intérieur du réservoir (1) étanche jusqu'à stabilisation de ladite pression,
- à déterminer à partir des valeurs enregistrées de la pression, au moins un des trois paramètres caractérisants suivants : un délai d'initiation de la réaction, un potentiel de neutralisation, une vitesse de réaction.

**2.** Méthode selon la revendication 1 caractérisée en ce que le délai d'initiation de la réaction est déterminé par la mesure du temps qui s'écoule entre le début du dépôt de l'acide liquide et l'instant de passage par une valeur minimale de la pression enregistrée.

**3.** Méthode selon la revendication 1 caractérisée en ce que la quantité de bases apportées par les additifs détergents contenus dans l'échantillon de lubrifiant étant connue, la pression enregistrée atteignant une valeur stable, le potentiel de neutralisation est déterminé par application de la formule suivante :

$$Ra = \frac{\Delta P}{Pth} \times 100$$

dans laquelle :

Ra    représente le potentiel de neutralisation en %,

$\Delta P$    représente la différence entre la valeur de pression enregistrée stabilisée et la valeur minimale de la pression enregistrée,

Pth    représente la pression théorique dans le réservoir (1) étanche, qui aurait été atteinte si la réaction avait été complète.

**4.** Méthode selon la revendication 1 caractérisée en ce que la vitesse de réaction est égale à la vitesse maximale d'augmentation de la pression enregistrée.

**5.** Méthode selon l'une des revendications 1 à 4 caractérisée en ce que le film de lubrifiant formé à l'intérieur du réservoir étanche présente une épaisseur comprise entre 20 et 150 microns.

**6.** Méthode selon l'une des revendications 1 à 5 caractérisée en ce que l'acide liquide est préférentiellement de l'acide sulfurique à 95%.

**7.** Méthode selon la revendication 6 caractérisée en ce que la quantité déterminée d'acide sulfurique à 95%, correspond à un excès d'acide compris entre 0,5% et 200 % et préférentiellement égal à 96,7%, par rapport à la quantité de bases apportée par les additifs détergents.

**8.** Dispositif pour la caractérisation de la capacité de neutralisation d'un lubrifiant contenant des additifs détergents renfermant une réserve de basicité, comportant un réservoir (1) étanche muni de moyens de maintien à une température constante, dans lequel on fait réagir les bases apportées par les additifs contenus dans un échantillon de masse connue de lubrifiant avec une quantité déterminée d'un acide liquide, au moins égale à la quantité stœchiométrique, caractérisé en ce qu'il comporte en plus :

- des moyens (13, 14) de formation d'un film, sur une paroi interne du réservoir (1) étanche à partir de l'échantillon de lubrifiant,

- des moyens (15) de dépôt de la quantité déterminée d'acide liquide à la surface du film dans un temps inférieur à 0,5 seconde,

- un capteur (8) de mesure de pression à l'intérieur du réservoir (1) étanche délivrant un signal de mesure de pression sur une sortie (9),

- un module (19) d'enregistrement relié à la sortie (9) du capteur (8) de pression pour enregistrer en fonction du temps les valeurs de la pression à l'intérieur du réservoir étanche,

- des moyens (22) de détermination d'au moins un des trois paramètres caractérisants suivants : un délai d'initiation de la réaction, un potentiel de neutralisation, une vitesse de réaction.

**9.** Dispositif selon la revendication 8 caractérisé en ce que les moyens (22) de détermination des paramètres caractérisants consistent en un module de visualisation d'une courbe représentative de la valeur de la pression enregistrée en fonction du temps, dans un système d'axes gradués, ledit module étant raccordé à une sortie (21) du module (19) d'enregistrement.

**10.** Dispositif selon la revendication 9 caractérisé en ce que le délai d'initiation de la réaction est déterminé à partir de la courbe représentative de la valeur de la pression enregistrée, par la mesure du temps qui s'écoule entre le début du dépôt de l'acide liquide et l'instant de passage par une valeur minimale de la pression enregistrée.

**11.** Dispositif selon la revendication 9 caractérisé en ce que la quantité de bases apportées par les additifs détergents contenus dans l'échantillon de lubrifiant étant connue, la pression enregistrée atteignant une valeur stable, le potentiel de neutralisation est déterminé par application de la formule suivante :

$$Ra = \frac{\Delta P}{Pth} \times 100$$

dans laquelle :

Ra    représente le potentiel de neutralisation en %,

$\Delta P$    représente la différence entre la valeur de pression enregistrée stabilisée et la valeur minimale de la pression enregistrée,

Pth    représente la pression théorique dans le réservoir (1) étanche, qui aurait été atteinte si la réaction avait été complète,

ΔP    est déterminée à partir de la courbe représentative de la valeur de la pression enregistrée.

**12.** Dispositif selon la revendication 9 caractérisé en ce que la vitesse de réaction, égale à la vitesse maximale d'augmentation de la pression enregistrée est déterminée à partir de la courbe représentative de la valeur de la pression enregistrée.

**13.** Dispositif selon la revendication 8 caractérisé en ce qu'il comporte en plus un indicateur (23) de début du dépôt d'acide liquide qui délivre sur une sortie (24) un signal de début de dépôt d'acide liquide, reliée au module (19) d'enregistrement et en ce que les moyens de détermination des paramètres caractérisants consistent un module électronique de calcul desdits paramètres, raccordé à une sortie (21) du module (19) d'enregistrement

**14.** Dispositif selon la revendication 13 caractérisé en ce que le module (25) électronique de calcul détermine le délai d'initiation de la réaction à partir de la valeur de la pression enregistrée et du signal délivré par l'indicateur (23) de début du dépôt d'acide liquide, par le calcul du temps qui s'écoule entre le début du dépôt de l'acide liquide et l'instant de passage par une valeur minimale de la pression enregistrée.

**15.** Dispositif selon la revendication 13 caractérisé en ce que la quantité de bases apportées par les additifs détergents contenus dans l'échantillon de lubrifiant étant connue, la pression enregistrée atteignant une valeur stable, le module (25) électronique de calcul détermine le potentiel de neutralisation par application de la formule suivante :

$$Ra = \frac{\Delta P}{Pth} \times 100$$

dans laquelle :

Ra    représente le potentiel de neutralisation en %,

ΔP    représente la différence entre la valeur de pression enregistrée stabilisée et la valeur minimale de la pression enregistrée,

Pth    représente la pression théorique dans le réservoir (1) étanche, qui aurait été atteinte si la réaction avait été complète,

ΔP    est calculée par le module (25) électronique de calcul à partir des valeurs de la pression enregistrée.

**16.** Dispositif selon la revendication 13 caractérisé en ce que le module (25) électronique de calcul détermine la vitesse de réaction, à partir des valeurs de la pression enregistrée, par le calcul de la vitesse maximale d'augmentation de la pression enregistrée.

**Claims**

**1.** Method of characterising the neutralising capacity of a lubricant containing detergent additives having excess basicity, consisting in reacting, in a tight reservoir (1), at constant temperature, the bases supplied by the additives contained in a sample of known mass of lubricant with a given quantity of a liquid acid, at least equal to the stoichiometric quantity, characterised in that it further consists in:

- forming, inside the tight reservoir (1), a film from the sample of lubricant,
- depositing the given quantity of liquid acid on the surface of the film within a period of less than 0.5 second,
- recording, as a function of time, the values of the pressure inside the tight reservoir (1) until the said pressure stabilises,
- determining from the recorded pressure values at least one of the following three characterising parameters: a reaction initiation time, a neutralising potential, a rate of reaction.

**2.** Method according to claim 1, characterised in that the reaction initiation time is determined by measuring the time which elapses between the start of the deposition of the liquid acid and the moment at which a minimum value of the recorded pressure is passed through.

**3.** Method according to claim 1, characterised in that, the quantity of bases supplied by the detergent additives contained in the sample of lubricant being known and the recorded pressure reaching a stable value, the neutralising potential is determined by applying the following formula:

$$Ra = \frac{\Delta P}{Pth} \times 100$$

in which:

Ra    represents the neutralising potential in %,

ΔP    represents the difference between the stabilised recorded pressure value and the minimum value of the recorded pressure,

Pth    represents the theoretical pressure in the tight reservoir (1), which would have been reached if the reaction had been complete.

**4.** Method according to claim 1, characterised in that the rate of reaction is equal to the maximum rate of increase of the recorded pressure.

5. Method according to any one of claims 1 to 4, characterised in that the film of lubricant formed inside the tight reservoir has a thickness of from 20 to 150 microns.

6. Method according to any one of claims 1 to 5, characterised in that the liquid acid is preferably 95 % sulphuric acid.

7. Method according to claim 6, characterised in that the given quantity of 95 % sulphuric acid corresponds to an excess of acid of from 0.5 % to 200 % and preferably equal to 96.7 %, relative to the quantity of bases supplied by the detergent additives.

8. Device for characterising the neutralising capacity of a lubricant containing detergent additives having excess basicity, comprising a tight reservoir (1) equipped with means for maintaining a constant temperature, in which the bases supplied by the additives contained in a sample of known mass of lubricant are reacted with a given quantity of a liquid acid, at least equal to the stoichiometric quantity, characterised in that it further comprises:

   - means (13, 14) for forming a film, on an inner wall of the tight reservoir (1) from the sample of lubricant,
   - means (15) for depositing the given quantity of liquid acid on the surface of the film within a period of less than 0.5 second,
   - a sensor (8) for measuring the pressure inside the tight reservoir (1), which delivers a pressure measuring signal at an output (9),
   - a recording module (19) connected to the output (9) of the pressure sensor (8) for recording, as a function of time, the values of the pressure inside the tight reservoir,
   - means (22) for determining at least one of the following three characterising parameters: a reaction initiation time, a neutralising potential, a rate of reaction.

9. Device according to claim 8, characterised in that the means (22) for determining the characterising parameters comprise a module for displaying a curve representing the value of the pressure recorded as a function of time, in a system of graduated axes, the said module being connected to an output (21) of the recording module (19).

10. Device according to claim 9, characterised in that the reaction initiation time is determined from the curve representing the value of the recorded pressure, by measuring the time which elapses between the start of the deposition of the liquid acid and the moment at which a minimum value of the recorded pressure is passed through.

11. Device according to claim 9, characterised in that, the quantity of bases supplied by the detergent additives contained in the sample of lubricant being known and the recorded pressure reaching a stable value, the neutralising potential is determined by applying the following formula:

$$Ra = \frac{\Delta P}{Pth} \times 100$$

in which:

   Ra    represents the neutralising potential in %,
   $\Delta P$    represents the difference between the stabilised recorded pressure value and the minimum value of the recorded pressure,
   Pth    represents the theoretical pressure in the tight reservoir (1), which would have been reached if the reaction had been complete,
   $\Delta P$    is determined from the curve representing the value of the recorded pressure.

12. Device according to claim 9, characterised in that the rate of reaction, which is equal to the maximum rate of increase of the recorded pressure, is determined from the curve representing the value of the recorded pressure.

13. Device according to claim 8, characterised in that it further comprises an indicator (23) of the start of the deposition of liquid acid, which indicator (23) delivers at an output (24), connected to the recording module (19), a signal indicating the start of the deposition of liquid acid, and in that the means for determining the characterising parameters comprise an electronic module for calculating the said parameters connected to an output (21) of the recording module (19).

14. Device according to claim 13, characterised in that the electronic calculating module (25) determines the reaction initiation time from the value of the recorded pressure and from the signal delivered by the indicator (23) of the start of the deposition of liquid acid, by calculating the time which elapses between the start of the deposition of the liquid acid and the moment at which a minimum value of the recorded pressure is passed through.

15. Device according to claim 13, characterised in that, the quantity of bases supplied by the detergent additives contained in the sample of lubricant being known and the recorded pressure reaching a stable value, the electronic calculating module (25) determines the neutralising potential by applying the following formula:

$$Ra = \frac{\Delta P}{Pth} \times 100$$

in which:

Ra      represents the neutralising potential in %,

$\Delta P$      represents the difference between the stabilised recorded pressure value and the minimum value of the recorded pressure,

Pth      represents the theoretical pressure in the tight reservoir (1), which would have been reached if the reaction had been complete,

$\Delta P$      is calculated by the electronic calculating module (25) from the values of the recorded pressure.

16. Device according to claim 13, characterised in that the electronic calculating module (25) determines the rate of reaction from the values of the recorded pressure, by calculating the maximum rate of increase of the recorded pressure.

**Patentansprüche**

1. Verfahren zur Kennzeichnung des Neutralisationsvermögens eines Schmiermittels, das Detergenzien mit einer Basizitätsreserve enthält, das darin besteht, dass man in einem dichten Behälter (1) bei konstanter Temperatur die Basen, die durch die Additive, die in einer Schmiermittelprobe von bekannter Masse enthalten sind, eingebracht werden, mit einer festgelegten Menge einer flüssigen Säure, die wenigstens der stöchiometrischen Menge entspricht, umsetzt, dadurch **gekennzeichnet**, dass es außerdem

    - in der Bildung eines Films aus der Schmiermittelprobe im Inneren des dichten Behälters (1),
    - in der Abscheidung der festgelegten Menge an flüssiger Säure auf der Filmoberfläche innerhalb eines Zeitraums von unter 0,5 Sekunden,
    - in der Aufzeichnung der Druckwerte im Inneren des dichten Behälters (1) in Abhängigkeit von der Zeit bis zur Stabilisierung des Drucks und
    - in der Ermittlung wenigstens eines der drei folgenden kennzeichnenden Parameter ausgehend von den aufgezeichneten Druckwerten besteht: Verzögerung des Reaktionsbeginns, Neutralisationspotential und Reaktionsgeschwindigkeit.

2. Verfahren nach Anspruch 1, dadurch **gekennzeichnet**, dass die Verzögerung des Reaktionsbeginns anhand der Zeit ermittelt wird, die zwischen dem Beginn der Abscheidung der flüssigen Säure und dem Moment des Hindurchtretens des aufgezeichneten Drucks durch einen Minimalwert vergeht.

3. Verfahren nach Anspruch 1, dadurch **gekennzeichnet**, dass, wenn die Menge der durch die in der Schmiermittelprobe enthaltenen Detergenzien eingebrachten Basen bekannt ist und der aufgezeichnete Druck einen konstanten Wert erreicht, das Neutralisationspotential durch Anwendung der folgenden Formel ermittelt wird:

$$Ra = \frac{\Delta P}{Pth} \times 100$$

wobei

Ra      das Neutralisationspotential in %,

$\Delta P$      die Differenz zwischen dem Wert des aufgezeichneten stabilisierten Drucks und dem Minimalwert des aufgezeichneten Drucks und

Pth      den theoretischen Druck im dichten Behälter (1), der, wenn die Reaktion abgeschlossen ist, erreicht wird,

bedeuten.

4. Verfahren nach Anspruch 1, dadurch **gekennzeichnet,** dass die Reaktionsgeschwindigkeit der maximalen Geschwindigkeit der Zunahme des aufgezeichneten Drucks entspricht.

5. Verfahren nach einem der Ansprüche 1 bis 4, dadurch **gekennzeichnet**, dass der im Inneren des dichten Behälters gebildete Schmiermittelfilm eine Dicke von 20 bis 150 µm aufweist.

6. Verfahren nach einem der Ansprüche 1 bis 5, dadurch **gekennzeichnet**, dass die flüssige Säure vorzugsweise 95%ige Schwefelsäure ist.

7. Verfahren nach Anspruch 6, dadurch **gekennzeichnet**, dass die festgelegte Menge an 95%iger Schwefelsäure einem Säureüberschuss von 0,5 bis 200 % und vorzugsweise von 96,7 %, bezogen auf die durch die Additive eingebrachte Basenmenge, entspricht.

8. Vorrichtung zur Kennzeichnung des Neutralisationsvermögens eines Schmiermittels, das Detergenzien mit einer Basizitätsreserve enthält, die einen dichten Behälter (1) umfasst, der mit Mitteln zur Aufrechterhaltung einer konstanten Temperatur ausgestattet ist, in dem man die Basen, die durch die Additive, die in einer Schmiermittelprobe von bekannter Masse enthalten sind, eingebracht werden, mit einer festgelegten Menge einer flüssigen Säure, die wenigstens der stöchiometrischen Menge entspricht, umsetzt, dadurch **gekennzeichnet**, dass sie außerdem noch

- Mittel (13, 14) zur Bildung eines Films auf einer Innenwand des dichten Behälters (1),
- Mittel (15) zur Abscheidung der festgelegten Menge an flüssiger Säure auf der Filmoberfläche innerhalb eines Zeitraums von unter 0,5 Sekunden,
- einen Fühler (8) zur Messung des Drucks im Inneren des dichten Behälters (1), der an einem Ausgang (9) ein Druckmesssignal abgibt,
- ein mit dem Ausgang (9) des Druckfühlers (8) verbundenes Aufzeichnungselement (19) zur Aufzeichnung der Druckwerte im Inneren des dichten Behälters in Abhängigkeit von der Zeit und
- Mittel (22) zur Ermittlung wenigstens eines der drei folgenden kennzeichnenden Parameter umfasst: Verzögerung des Reaktionsbeginns, Neutralisationspotential und Reaktionsgeschwindigkeit.

9. Vorrichtung nach Anspruch 8, dadurch **gekennzeichnet**, dass die Mittel (22) zur Ermittlung der kennzeichnenden Parameter in einem Bauelement zur Sichtbarmachung einer repräsentativen Kurve des aufgezeichneten Druckwerts in Abhängigkeit von der Zeit in einem System aus eine Einteilung aufweisenden Achsen bestehen, wobei dieses Bauelement mit einem Ausgang (21) des Aufzeichnungselements (19) verbunden ist.

10. Vorrichtung nach Anspruch 9, dadurch **gekennzeichnet**, dass die Verzögerung des Reaktionsbeginns ausgehend von der repräsentativen Kurve des aufgezeichneten Druckwerts anhand der Zeit ermittelt wird, die zwischen dem Beginn der Abscheidung der flüssigen Säure und dem Moment des Hindurchtretens des aufgezeichneten Drucks durch einen Minimalwert vergeht.

11. Vorrichtung nach Anspruch 9, dadurch **gekennzeichnet**, dass, wenn die Menge der durch die in der Schmiermittelprobe enthaltenen Detergenzien eingebrachten Basen bekannt ist und der aufgezeichnete Druck einen konstanten Wert erreicht, das Neutralisationspotential durch Anwendung der folgenden Formel ermittelt wird:

$$Ra = \frac{\Delta P}{Pth} \times 100$$

wobei

Ra das Neutralisationspotential in %,
ΔP die Differenz zwischen dem Wert des aufgezeichneten stabilisierten Drucks und dem Minimalwert des aufgezeichneten Drucks und
Pth den theoretischen Druck im dichten Behälter (1), der erreicht wird, wenn die Reaktion abgeschlossen ist, darstellen, und
ΔP ausgehend von der repräsentativen Kurve des aufgezeichneten Druckwerts anhand der Zeit ermittelt wird.

12. Vorrichtung nach Anspruch 9, dadurch **gekennzeichnet**, dass die Reaktionsgeschwindigkeit, die der maximalen Geschwindigkeit der Zunahme des aufgezeichneten Drucks entspricht, ausgehend von der repräsentativen Kurve des aufgezeichneten Druckwerts anhand der Zeit ermittelt wird.

13. Vorrichtung nach Anspruch 8, dadurch **gekennzeichnet**, dass sie außerdem noch eine Vorrichtung (23) zur Anzeige des Beginns der Abscheidung der flüssigen Säure umfasst, die an einem Ausgang (24) ein Signal für den Beginn der Abscheidung der flüssigen Säure abgibt und mit dem Aufzeichnungselement (19) verbunden ist, und die Mittel zur Ermittlung der kennzeichnenden Parameter in einem elektronischen Bauelement zur Berechnung dieses Parameters, das mit einem Ausgang (21) des Aufzeichnungselements (19) verbunden ist, bestehen.

14. Vorrichtung nach Anspruch 13, dadurch **gekennzeichnet**, dass das elektronische Berechnungsbauelement (25) die Verzögerung des Beginns der Reaktion ausgehend vom aufgezeichneten Druckwert und dem von der Vorrichtung (23) für die Anzeige des Beginns der Abscheidung der flüssigen Säure abgegebenen Signal durch Berechnung der Zeit ermittelt, die zwischen dem Beginn der Abscheidung der flüssigen Säure und dem Moment des Hindurchtretens des aufgezeichneten Drucks durch einen Minimalwert vergeht.

15. Vorrichtung nach Anspruch 13, dadurch **gekennzeichnet**, dass, wenn die Menge der durch die in der Schmiermittelprobe enthaltenen Detergenzien eingebrachten Basen bekannt ist und der aufgezeichnete Druck einen konstanten Wert erreicht, das elektronische Berechnungsbauelement das Neutralisationspotential durch Anwendung der folgenden Formel ermittelt:

$$Ra = \frac{\Delta P}{Pth} \times 100$$

wobei

Ra das Neutralisationspotential in %,
ΔP die Differenz zwischen dem Wert des aufgezeichneten stabilisierten Drucks und dem Minimalwert des aufgezeichneten Drucks,

Pth den theoretischen Druck im dichten Behälter (1), der erreicht wird, wenn die Reaktion abgeschlossen ist, darstellen, und

$\Delta P$ vom elektronischen Berechnungsbauelement (25) ausgehend von den aufgezeichneten Druckwerten berechnet wird.

16. Vorrichtung nach Anspruch 13, dadurch **gekennzeichnet**, dass das elektronische Berechnungsbauelement die Reaktionsgeschwindigkeit ausgehend von den aufgezeichneten Druckwerten durch Berechnung der Maximalgeschwindigkeit der Zunahme des aufgezeichneten Drucks ermittelt.

FIG.1

FIG.2

FIG.3

FIG.4

FIG.5

EP 0 930 503 B1